# EUROPEAN PATENT APPLICATION

(11) **EP 4 557 263 A1**
(43) Date of publication of application: **21.05.2025**
(21) Application number: 23839668.3
(22) Date of filing: 12.07.2023
(51) Int. Cl.: G09B 19/24, G01N 21/359, A61B 5/06

(54) **DEVICE AND METHOD FOR OBSERVING BLOOD VESSEL MODEL**

(30) Priority: 12.07.2022 JP 2022111530
(71) Applicant: Fain-Biomedical Inc., Nagoya-shi, Aichi 451-0042 (JP)
(72) Inventor: IKEDA Seiichi, Okayama-shi, Okayama 701-1221 (JP); IKEDA Moritaka, Okayama-shi, Okayama 701-1221 (JP)
(74) Representative: Kondrat, Mariusz
(86) International application number: PCT/JP2023/025803
(87) International publication number: WO 2024/014496

(57) **Abstract**

Provided are an observation device and an observation method capable of simultaneously observing a fluoroscopic image of a catheter inserted into a blood vessel model and the blood vessel model itself without using an X-ray. A blood vessel model observation device that observes a shape of a blood vessel model and a catheter inserted into the blood vessel model, the blood vessel model including a substance disposed inside having near-infrared light absorption characteristics different from that of a constituent material of the blood vessel model, and the catheter including a sheath and wires inserted into the sheath, the blood vessel model observation device including: a light source that emits near-infrared light; a light amount adjustment device that adjusts a light amount of the near-infrared light; a camera; and a display that displays an image captured by the camera.

## Description

### TECHNICAL FIELD

The present invention relates to a device and a method for observing a blood vessel model and a catheter inserted into said blood vessel model.

### BACKGROUND ART

In evaluation of a catheter to be inserted into a blood vessel, and in technical training and technical evaluation of an intravascular surgery, it is necessary to observe a shape of a blood vessel model (hereinafter, referred to as "blood vessel shape") and a state of a catheter inserted into said blood vessel model.

In order to observe the blood vessel shape and the state of the catheter inserted into the blood vessel model, a visible light camera or an X-ray projection device is often used in addition to direct visual observation.

By forming a blood vessel model with a transparent material and filling a lumen portion (hereinafter, referred to as "lumen region") of said blood vessel model with a liquid colored with a pigment or the like, a method of visually observing the blood vessel shape and the catheter inserted into the blood vessel model or observing the blood vessel shape and the catheter with a visible light camera is simple. However, in this case, since the catheter cannot be seen through and the position and state of a guide wire or the like constituting the catheter cannot be confirmed, the reproducibility of the intravascular surgery is low. This problem can be solved by using an X-ray, but it is not easy to use the X-ray from the viewpoint of radiation exposure and management.

In general, a catheter has a multilayer structure in which wires (balloon, coil, stent or the like having a therapeutic function, drug (liquid such as contrast medium or thrombolytic agent), and other sheath) are inserted into a thin tubular sheath, and it is not possible to detect a distal end position and a state of the wires inserted into the sheath in a multilayer manner by observation using visible light.

In actual treatment using a catheter, information on the distal end position of the wires in the sheath and the state thereof is required. Furthermore, in the practical catheter, a material that does not transmit an X-ray (X-ray opaque marker) is attached to a distal end, an intermediate portion, or the like of the sheath or the wires in order to clearly indicate a specific position of the sheath or the wires, and it is possible to confirm a relative position of each of the sheath or the wires in the X-ray projection device used at the time of treatment.

An observation method combining a fluorescence observation technique and an image processing technique has been proposed in order to enable observation of a distal end position and a state of wires in a sheath while avoiding exposure to X-rays (Non-Patent Literature 1).

Furthermore, Patent Literature 1 proposes a lubricating liquid that ensures lubricity between a blood vessel model and a catheter.

### CITATIONS LIST

### PATENT LITERATURE

Patent Literature 1: JP 5992031 B2

NON-PATENT LITERATURE

Non-Patent Literature 1: Kazuaki Fukasaku et al. "Endovascular treatment training is enabled at anytime and anywhere - Developing simulation of endovascular treatment that does not expose doctors to X-rays -", [online], February 19, 2022, The 51st Japan Society for Neuroradiology https://www.u-ryukyu.ac.jp/news/32074/

### SUMMARY OF INVENTION

### TECHNICAL PROBLEMS

According to the observation method introduced in Non-Patent Literature 1, it is necessary to attach a fluorescent material to each portion of the sheath and wires of the catheter to be visualized. In other words, the actual catheter cannot be used as it is.

Moreover, in order to visualize the blood vessel model, it is necessary to introduce a contrast medium (liquid containing a fluorescent dye) into the liquid filling the lumen region of the blood vessel model.

For example, in a case where the blood vessel model is formed of a gel material made of polyvinyl alcohol or the like, there is a possibility that the contrast medium exudes into a blood vessel model forming material or adheres to an inner surface of the blood vessel model, and thus it is difficult to withstand long-term use.

On the other hand, in a case where the blood vessel model is formed of a crosslinked polymer material such as silicone rubber, it is necessary to fill the lumen of the blood vessel model with a lubricating liquid in order to ensure lubricity between the catheter and the blood vessel model (see Patent Literature 1). When the contrast medium is mixed with the lubricating liquid, the lubricating function of the lubricating liquid may be deteriorated.

### SOLUTIONS TO PROBLEMS

As a result of intensive studies to solve the above problems, the present inventors have found that by using near-infrared light having higher substance permeability than visible light and variously changing a light absorption rate depending on the material and wavelength as compared with visible light, the blood vessel shape and the state inside the catheter can be simultaneously or selectively visualized without using a contrast medium or the like. That is, it is not necessary to attach the contrast medium or the fluorescent material required in the prior art to the sheath or the wires of the catheter.

The near-infrared absorption rate varies depending on the type of substance and the wavelength of near-infrared light to be used. According to this characteristic, in near-infrared spectroscopy, a change in absorption rate in a near-infrared region is measured, and a component of a substance is specified by comparing a measurement result with a spectrum table showing absorption characteristics. Furthermore, in many substances, the absorption rate is greatly different in the visible light region and the near-infrared region. According to this characteristic of the near-infrared light, a liquid containing a dye or the like that appears opaque in the visible light region can be observed transparently in the near-infrared region, and various soft polymers such as silicone rubber that appears transparent in the visible light region, various hard polymers such as an acrylic resin, and various liquids such as water and oil can be observed (hereinafter, referred to as "near-infrared spectroscopic observation") as different colors (shades) in the near-infrared region without using a contrast agent or the like. Moreover, since the near-infrared absorption rate (that is, near-infrared transmittance) of each substance is variously changed by changing the wavelength in the near-infrared region, it is possible to identify a difference in shape and material of each part of the composite structure including different substances by using this characteristic.

The inventors of the present invention have found that, by performing the near-infrared spectroscopic observation due to this feature of near-infrared light, it is possible to observe each part of the blood vessel model, which is entirely transparent in the visible light region including the inside and outside, as an image in which shape and a difference in material can be identified as a different color (shade) without using a contrast medium or the like. Furthermore, it has been found that by changing the wavelength and light amount of the near-infrared light to be used (the intensity of a light source and the exposure setting of an observation device), the balance of shades of each part of the blood vessel model can be changed without applying a physical operation to the observation target. Furthermore, it has been found that the blood vessel shape can be selectively visualized or eliminated while the fluoroscopic image of the catheter is visualized on an observation screen by changing the wavelength and light amount of the near-infrared light.

On the other hand, similarly to the X-ray, the near-infrared light has a property of easily transmitting various materials, and thus, it is possible to perform fluoroscopic observation (hereinafter, referred to as "near-infrared fluoroscopic observation") of an object. As a simpler and safer method than X-ray observation, it is used for non-destructive inspection of semiconductors and the like, inspection of foreign matters in liquids, and the like.

In the observation with visible light, the blood vessel model is observed stereoscopically (3D), but according to the near-infrared fluoroscopic observation, a planar (2D) image like a shadow picture is obtained due to its permeability. Such a planar (2D) image is close to an X-ray fluoroscopic image obtained as in the catheter intravascular surgery, and is suitable for reproducing the simulation of the same surgery. Moreover, even in a case where the respective parts included in the blood vessel model intersect stereoscopically, that is, the parts position in front and behind at an irradiation direction of near-infrared light, a planar (2D) fluoroscopic image similar to the X-ray observation image can be obtained.

The catheter of an actual machine has a thin film-like multilayer structure as a common feature, but the constituent material of the sheath and the film thickness thereof are not uniform depending on the type (as a constituent material of the sheath, various soft resins such as polyester, polyethylene, polyamide (nylon), polyurethane, and silicone rubber are used). Furthermore, metal structures such as a mesh and an X-ray opaque marker, mechanical structures such as a balloon and a coil detachment mechanism, and the like are complicatedly incorporated in the catheter and the wires. For the catheter having such characteristics, the inventors have found that by performing observation incorporating two aspects, the near-infrared fluoroscopic observation and the near-infrared spectroscopic observation, the catheter can be reproduced as an image close to an X-ray observation image at the time of actual surgery and further as a function-expanded observation image including information that cannot be obtained by the X-ray observation.

By using the near-infrared light, it is possible to perform fluoroscopic observation of the catheter and obtain an observation image capable of identifying a difference in structure and material thereof, and further, by changing the wavelength and light amount of the near-infrared light to be used, it is possible to change the shading balance of the observation image without applying a physical operation to the blood vessel model. For example, it is possible to select a specific region of the blood vessel model to be observed, change or reverse the shading balance of the selected specific region, and visualize or eliminate the specific region.

A first aspect of the present invention is defined as follows.

An observation device that observes a blood vessel model as an observation target into which a catheter is inserted, the blood vessel model including a substance disposed inside having near-infrared light absorption characteristics different from that of a constituent material of the blood vessel model, and the catheter including a sheath and wires inserted into the sheath, the observation device including: a first light source that emits first near-infrared light; a first light reception unit that can receive the first near-infrared light; a first image generation unit that generates a first image based on the first near-infrared light captured by the first light reception unit; and a display that displays the first image.

According to the blood vessel model observation device of the first aspect defined as described above, since the substance disposed in the blood vessel model has near-infrared light absorption characteristics different from that of the constituent material of the blood vessel model, it is possible to visually recognize a boundary between a blood vessel wall cross-sectional region (hereinafter, referred to as a "wall cross-sectional region") of the blood vessel model and the substance in near-infrared spectroscopic observation. At this time, since the catheter inserted into the blood vessel model has permeability different from the constituent material of the blood vessel model and the substance with respect to near-infrared light, the catheter can be observed distinguishably from the blood vessel model and the substance.

A second aspect of the present invention is defined as follows.

An observation method of an observation target using the observation device according to the first aspect, the observation method including:
causing the first light source to emit the first near-infrared light toward the observation target, and causing the first light reception unit to receive the first near-infrared light transmitted through the observation target;
causing the first image generation unit to generate the first image based on the first near-infrared light received by the first light reception unit;
causing the display to display the first image; and
adjusting a light amount of the first near-infrared light used for generating the first image as necessary to enable simultaneous visual recognition of the sheath of the catheter, the wires in the sheath, and the blood vessel model.

As described in the first aspect, the blood vessel model and the catheter inserted into the blood vessel model can be distinguished and observed by the near-infrared spectroscopic observation. By adjusting the light amount of the first near-infrared light used for generating the first image as necessary, it is possible to display the fluoroscopic image of the catheter together with the blood vessel model on the image of the display. That is, near-infrared light fluoroscopic observation can be performed by adjusting the light amount of the near-infrared light.

Here, as necessary means that if the blood vessel model and the catheter constituting the observation target are standardized, the light amount is adjusted in advance, so that the light amount does not need to be adjusted at the time of observation (hereinafter, the same applies to this specification).

Since it is a premise of the catheter simulation that the sheath of the catheter, the wires in the sheath, and the blood vessel model can be simultaneously visually recognized, the observation method of the blood vessel model defined in the second aspect is practical. By adjusting the light amount of the near-infrared light, it is also possible to selectively display only an outline image (non-fluoroscopic image) of the catheter, the outline image of the catheter and an image of the blood vessel model, only a fluoroscopic image of the catheter, and only the blood vessel model on the image of the display.

In the above, as an adjustment device that adjusts the light amount of the near-infrared light, a device that is attached to a light source of near-infrared light and adjusts a light amount of near-infrared light emitted from the light source, a diaphragm that is attached to a light reception unit such as a camera and adjusts a light amount of light collected by the camera, a device that processes an image captured by the camera and adjusts an amount of light, and the like can be used.

A third aspect of the present invention utilizes near-infrared reflection observation.

The reflection characteristics of near-infrared light vary depending on a material irradiated with the near-infrared light. For example, there is a difference in reflection characteristics between iron (stainless steel) and platinum.

As the wires constituting the catheter, there is a wire in which platinum is attached to the middle of a stainless wire to form an X-ray opaque marker. Since both stainless steel and platinum do not transmit near-infrared light, neither near-infrared spectroscopic observation nor near-infrared fluoroscopic observation is effective for such a wire.

On the other hand, since stainless steel and platinum have different near-infrared reflection characteristics, by emitting near-infrared light from a second near-infrared light source to condense reflected light from an object (in the above example, a wire including a platinum marker) on a light reception unit, stainless steel that is a base metal of the wire and platinum that is a marker can be observed distinguishably. This observation can be performed together with the near-infrared spectroscopic observation and the near-infrared fluoroscopic observation described in the second aspect.

Therefore, a third aspect of the present invention is defined as follows. That is,
the observation device according to the first aspect, further including: a second light source that emits second near-infrared light; a second light reception unit that can receive the second near-infrared light; a second image generation unit that generates a second image based on the second near-infrared light captured by the second light reception unit; and an image synthesis unit,
in which the image synthesis unit superimposes the second image on the first image to form a composite image, and
causes the display to display the composite image.

An observation method by the observation device of the third aspect defined as described above is as follows.

An observation method of an observation target using the observation device according to the third aspect, the observation method including:
causing the first light source to emit the first near-infrared light toward the observation target, and causing the first light reception unit to receive the first near-infrared light transmitted through the observation target;
causing the first image generation unit to generate the first image based on the first near-infrared light received by the first light reception unit;
causing the second light source to emit the second near-infrared light toward the observation target, and causing the second light reception unit to receive the second near-infrared light reflected by the observation target;
causing the second image generation unit to generate the second image based on the second near-infrared light received by the second light reception unit;
causing the image synthesis unit to generate a composite image in which the first image and the second image are superimposed; and
causing the display to display the composite image.

Here, by adjusting a light amount of the first near-infrared light used for generating the first image as necessary, and/or adjusting a light amount of the second near-infrared light used for generating the second image as necessary, the sheath of the catheter, the wires in the sheath, an X-ray opaque marker of the wires, and the blood vessel model can be visually recognized at the same time.

The observation device according to a fifth aspect of the present invention is defined as follows. That is, the observation device according to the third aspect, further including:
a grayscale image generation unit that gray-scales the first image and the second image; and
an image inversion unit that generates an image 2-1 by inverting gradation of a grayscale image of the second image.

An observation method by the observation device of the fifth aspect defined as described above is as follows.

An observation method using the observation device according to the fifth aspect,
the observation method including:
causing the grayscale image generation unit to grayscale the first image and the second image;
causing the image inversion unit to invert gradation of the gray-scaled second image to form the image **2-1;** and
causing the image synthesis unit to superimpose the gray-scaled first image and the image 2-1, and synthesize them by calculating value of each pixel on the images.

The observation device according to a seventh aspect of the present invention is defined as follows.

That is, the observation device according to the fifth aspect, further including:
a first polarization section that polarizes the first near-infrared light in a first direction;
a second polarization section that polarizes the second near-infrared light in a second direction; and
a single housing that accommodates the first light reception unit and the second light reception unit, in which the first light reception unit can receive the first near-infrared light polarized in the first direction, and the second light reception unit can receive the second near-infrared light polarized in the second direction,
the first image generation unit generates the first image based on the first near-infrared light polarized in the first direction, and
the second image generation unit generates the second image based on the second near-infrared light polarized in the second direction.

An observation method by the observation device according to the seventh aspect defined as described above is as follows.

An observation method of an observation target using the observation device according to the seventh aspect, the observation method including:
causing the grayscale image generation unit to grayscale the first image and the second image;
causing the image inversion unit to invert gradation of the gray-scaled second image to form the image 2-1; and
causing the image synthesis unit to superimpose the gray-scaled first image and the image 2-1, and synthesize them by calculating value of each pixel on the images.

In the above description, the wavelengths of the near-infrared light from the first light source and the second light source are not 1300 nm to 1550 nm, and near-infrared light having a wavelength that is less affected by absorption by water can be used. Since an amount of water through which the first near-infrared light from the first light source are transmitted is different from an amount of water through which the second near-infrared light from the second light source are transmitted, in a case where near-infrared light having a wavelength that is greatly affected by light absorption are used, it is difficult to clearly display the transmitted image by the first light source and the reflected image by the second light source at the same time.

It is preferable to form the second light source with an LED of 1300 nm or less which is industrially easily available and has a relatively small influence of absorption by water.

In the above, it is preferable to use monochromatic light for the first and second near-infrared light. By installing the second near-infrared light in multiple positions and radiating it toward the observation target from multiple directions, the visibility of the X-ray opaque marker of the wires can be stably observed regardless of the position. Instead of installing the second near-infrared light in multiple positions, a mirror or a half mirror can be used in combination. By using a mirror or a half mirror, the number of the installation positions of the second near-infrared light can be reduced. In particular, in a case where the observation target is observed in a state of being immersed in the liquid, when the second near-infrared light is emitted toward the observation target, the second near-infrared light is reflected (including total reflection)/refracted by the surface of the liquid or the surface of the liquid storage container, so that irradiation from a specific direction is difficult or impossible. In such a case, this problem can be avoided by installing a mirror or a half mirror in the liquid, and irradiating the observation target with the second near-infrared light after reflecting the second near-infrared light. A mirror or a half mirror can be included as a part of the structure of the observation target including the blood vessel model regardless of the presence or absence of immersion in the liquid. In addition to reducing restrictions on the installation position of the second near-infrared light source, functions such as adjustment of a polarization state (inversion of the polarization state, etc.) can also be provided.

A plurality of monochromatic light having different wavelengths may be used for the second near-infrared light. In this case, each monochromatic light is polarized in different states and emitted toward the observation target, and the second light reception unit or the image synthesis unit synthesizes them optically or by numerical calculation, whereby the visibility of the X-ray opaque marker of the wires can be enhanced in near-infrared reflection observation.

In a near-infrared light having a wide wavelength (for example, near-infrared light lamp), it is necessary to adjust chromatic aberration depending on the wavelength. By using a lens having a focus shift correction function in the near-infrared region, the focal point of the lens can be set to the same position for different wavelengths. According to this method, it is not necessary to adjust the chromatic aberration for each wavelength, and it is possible to simplify the device configuration and improve the workability at the time of use. By using a polarization camera provided with a polarizing filter in a specific direction for each pixel, the first light reception unit and the second light reception unit can be integrated into one camera. According to this method, it is not necessary to adjust the relative position and the enlargement ratio of the image, and it is possible to simplify the device configuration and improve the workability at the time of use.

Here, the monochromatic near-infrared light includes near-infrared light emitted from the LEDs constituting the light source. Since the wavelength of near-infrared light from the LED light source is based on the band gap of the LED, when the band gap has a width due to impurities contained in the LED, near-infrared light having a wavelength width corresponding to the width is emitted from the LED light source.

The near-infrared light emitted from the near-infrared lamp can be monochromatized through a filter that transmits light in a selected wavelength.

### BRIEF DESCRIPTION OF DRAWINGS

FIG. 1 is a spectrum illustrating absorption characteristics of near-infrared light by water and silicone rubber.
FIG. 2 is a schematic diagram illustrating an observation device of the present invention.
FIG. 3 illustrates an observation image with visible light in FIG. 3A, and illustrates an observation image when near-infrared light of 1450 nm is used in FIG. 3B.
FIG. 4 is a graph illustrating a relationship among a particle radius, a wavelength, and Rayleigh observation.
FIG. 5 illustrates an observation image according to an embodiment of the present invention.
FIG. 6 illustrates an observation image by near-infrared reflection observation.
FIG. 7 is a block diagram illustrating an observation device according to another embodiment of the present invention.
FIG. 8 illustrates an example of a first image obtained from first near-infrared light in the device of FIG. 7.
FIG. 9 illustrates an example of a second image obtained from second near-infrared light in the device of FIG. 7.
FIG. 10 illustrates an example of an image 2-1 in which color tone of the second image is inverted.
FIG. 11 illustrates a composite image of the first image and the image 2-1.
FIG. 12 illustrates an image in which an X-ray opaque marker is emphasized by performing density correction on the image of FIG. 11.
FIG. 13 is a block diagram illustrating an observation device according to another embodiment of the present invention.
FIG. 14 illustrates an example of an image output from the device of FIG. 13.

### DESCRIPTION OF EMBODIMENTS

Near-infrared light generally refers to light having a wavelength of 780 nm to 2500 nm.

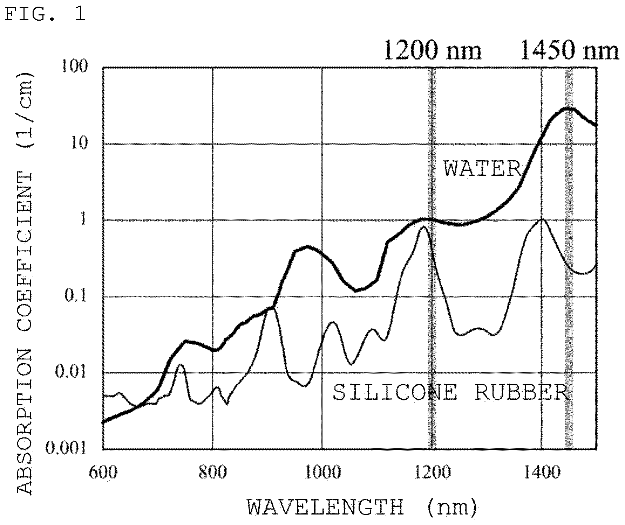

The absorption rate of near-infrared light changes according to the wavelength and the substance (see FIG. 1). It can be seen that the infrared absorption rate of water greatly changes according to the wavelength in the near-infrared region.

The lumen of the blood vessel model is often filled with a liquid in order to imitates blood, and as such a liquid, an aqueous solution in which a lubricating component is mixed as necessary with a liquid containing water as a main component similarly to blood is generally used. In order to simultaneously and effectively perform both near-infrared fluoroscopic observation and near-infrared spectroscopic observation, 1450 nm was selected as a wavelength at which the absorption rate of water was significantly high in the near-infrared region and which was easily differentiated from the absorption rate of air, silicone rubber, or the like, and observation of the blood vessel model and the catheter inserted into the blood vessel model was performed.

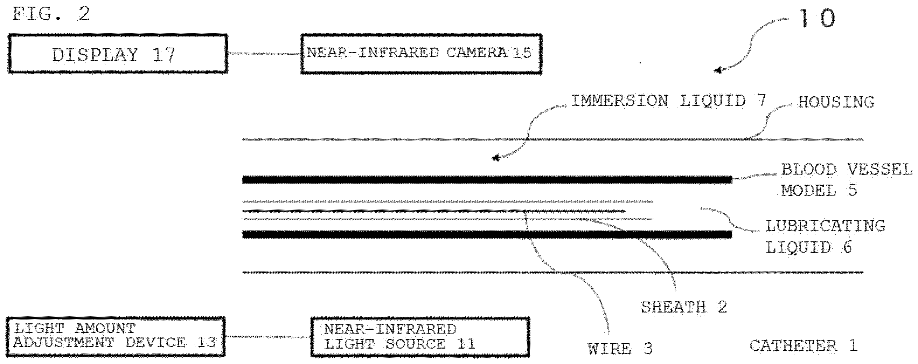

FIG. 2 illustrates a structure of an observation device 10.

Reference sign 1 in the drawing denotes a catheter. The catheter 1 includes a sheath 2 and wires 3 inserted into the sheath 2. Reference sign 5 denotes a blood vessel model, and a lumen of the blood vessel model 5 is filled with a lubricating liquid 6. The lubricating liquid may be circulated to impart fluidity to the lubricating liquid. The periphery of the blood vessel model 5 is filled with an immersion liquid 7 containing no lubricating component.

The observation device 10 includes a first near-infrared light source 11, a light amount adjustment device 13, a first near-infrared camera 15, and a display 17.

The blood vessel model is made of silicone rubber provided by FAIN-Biomedical Inc., and has a membrane thickness of 0.3 mm to 3.0 mm and a lumen of 1 mm to 15 mm. The lumen region of the blood vessel model is filled with a lubricating liquid in which a lubricating component is mixed with water. This lubricating liquid is a substance having near-infrared light absorption characteristics different from the constituent material of the blood vessel model.

As a catheter, an Excelsior microcatheter manufactured by Stryker Japan K.K. was used. As the wires, a GDC coil manufactured by Stryker Japan K.K. was used.

As the first near-infrared light source 11, an infrared illumination (model number: TH2-100X100IR145) manufactured by CCS Inc. was used. As the light amount adjustment device 13, a power supply (model number: PD-3024) having a function of adjusting an output of the light source 11, that is, a light amount of near-infrared light emitted from the light source 11 was used. As the near-infrared camera 15, a near-infrared camera (model number: ABA-013VIR) manufactured by Aval Data Corporation and a polarization camera (model number: XCG-CP510) manufactured by Sony Corporation were used, and as the camera lens, a near-infrared compatible lens (model number: LM35HC-SW and model number: LM35HC-VIS-SW) manufactured by Kowa Optronics Co., Ltd. was used.

As illustrated in FIG. 2, an observation target is set between the light source 11 and the camera 15. Near-infrared light having a wavelength of 1450 nm is emitted from the light source 11, and the light amount thereof is adjusted by the light amount adjustment device 13. The near-infrared light emitted from the light source 11 to the blood vessel model 5 transmits through the blood vessel model 5, is received by the camera 15, and an image thereof is displayed on the display 17.

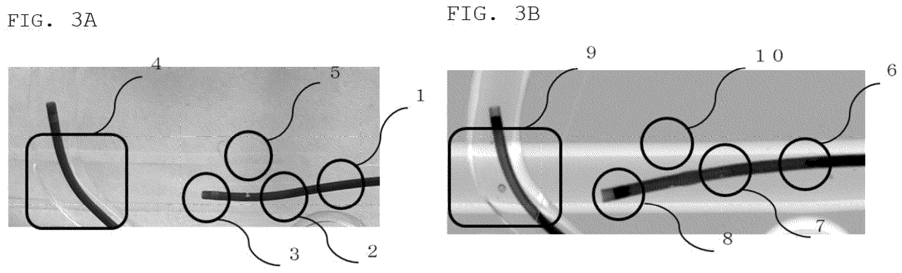

FIG. 3(A) illustrates an image obtained by monochromatizing an observation image of a blood vessel model and a catheter inserted into said blood vessel model observed by a visible light optical camera in the above setting. Furthermore, FIG. 3(B) illustrates an observation image of the blood vessel model and the catheter inserted into the blood vessel model of FIG. 3(A) which were observed by the camera 15 (the diaphragm of the lens attached to the camera was set to F2.8) using the light source 11 of 1450 nm (the light amount was set to the maximum in the light amount adjustment device attached to the light source).

The distal end positions of the wires disposed inside the catheter can be observed from reference signs 6, 7, 8, and the like in FIG. 3(B). According to the study of the present inventors, by adjusting the amount of light, a structure such as a metal mesh incorporated in the catheter could also be observed via fluoroscopy. The state and internal structure in the sheath of the catheter cannot be confirmed by the visible light observation illustrated in FIG. 3(A) (reference sign 3 is an appearance image of an X-ray opaque marker exposed on the surface of the sheath). Furthermore, the following can be understood from comparison between reference sign 10 and reference sign 5 in the figures. In the observation with visible light, the lumen region, the wall cross-sectional region, and the region around the blood vessel of the blood vessel model appear to be the same color (transparent). In the observation with near-infrared light, each of the above regions can be clearly identified by shading.

As can be seen from the reference sign 9 in the figure, even when a plurality of blood vessel branches included in the blood vessel model three-dimensionally intersect, visibility of both blood vessels is not reduced, and both blood vessels can be clearly observed as planar (2D) images. In the image by visible light, the outline of the blood vessel lumen is partially visually recognized as indicated by reference sign 4, but this outline is not an image in which the wall cross-sectional region is observed as indicated by reference sign 9, but is a reflected image generated by reflection of ambient light on the surface of the lumen region or the outer surface of the blood vessel model. Since this reflected image is visually recognized in a stereoscopic (3D) manner, a visualized image of the blood vessel lumen visualized by this method is different from a planar (2D) image obtained by X-ray observation.

Since the reflected image appears as a shading distribution on the lumen region, visibility of the lumen region decreases. Compared with the method of visualizing the wall cross-sectional area of the present invention, it is not preferable.

In the method using visible light, a part or the whole of the blood vessel model is viewed stereoscopically (3D) by reflection of ambient light or the like, and all opaque observation targets including catheters and wires are observed stereoscopically (3D).

Since the near-infrared light is not visually recognized (even if the light amount is increased, the near-infrared light is not dazzled), the use of the near-infrared light does not hinder the operation of the operator at all. In other words, even if the light amount of the near-infrared light source is arbitrarily adjusted so as to obtain the optimum observation condition, no burden is put on the operator.

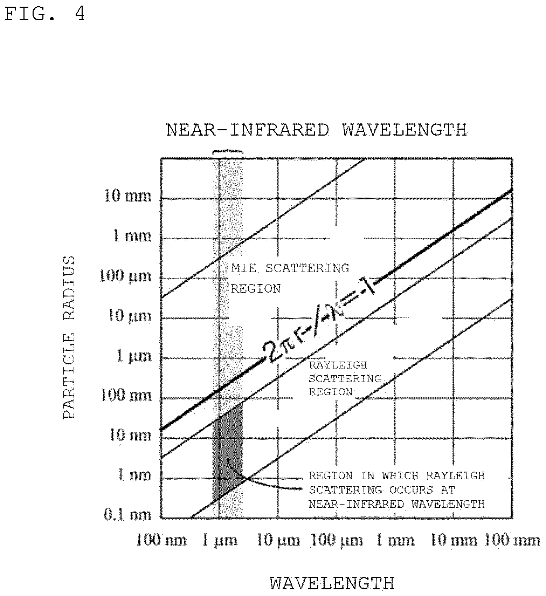

In the above description, the water-soluble polymer was dissolved in the lubricating liquid 6 filling the inside of the blood vessel model 5 and the immersion liquid 7 filling the periphery of the blood vessel model 5. Such a water-soluble polymer is considered to be particles having a diameter of several nm in an aqueous solution, and causes Rayleigh scattering as illustrated in FIG. 4. As a result, the near-infrared light from the light source 11 are scattered in the liquid, and the observation target is efficiently irradiated with the near-infrared light. Furthermore, if such a liquid is used, scattering occurs when the near-infrared light passes through the surface of the blood vessel model or is reflected on the outer surface, and image information such as a surrounding environment image included in the irradiated near-infrared light disappears. In addition to this method, it is also possible to scatter near-infrared light inside the structure of the blood vessel model to obtain a similar effect by mixing particles that cause Rayleigh scattering in the material forming the blood vessel model.

As a result, as compared with the case of using a liquid that does not cause Rayleigh scattering, each part of the obtained observation image was a more planar (2D) image close to the observation image by X-ray. This effect was particularly remarkably confirmed in the wall cross-sectional region of the blood vessel model, and when a liquid that causes Rayleigh scattering was used, the wall cross-sectional region was observed as a region of uniform color without pattern. On the other hand, in a liquid that does not cause Rayleigh scattering, an image of the surrounding environment or the like was projected on the wall cross-sectional region to generate shading or a pattern, and there were cases in which the region was observed slightly three-dimensionally.

As can be seen from FIG. 1, in the near-infrared region having a wavelength of 1300 nm to 1550 nm, the infrared absorption rate by water is higher than that in the region having a wavelength of 780 nm to 1200 nm. The infrared ray absorption characteristics of silicone rubber vary depending on the type, but silicone rubber (model number: SYLGARD 182 manufactured by The Dow Chemical Company) having the characteristics illustrated in FIG. 1 is lower than water in a main wavelength region of near-infrared light (in particular, when the wavelength is 1200 nm, the absorptivity is close to that of water, and when the wavelength is 1450 nm, the absorptivity is significantly higher than that of water).

According to the study of the present inventors, when near-infrared light having a wavelength of 1300 nm to 1550 nm is used, there is a difference in infrared ray absorptivity of a polymer material such as silicone rubber or acrylic resin or a gas such as air with respect to water disposed in an inner cavity region of a blood vessel model, and thus, near-infrared spectroscopic observation can be easily performed, and the inner cavity region can be clearly visualized. Note that, since the near-infrared absorption rate of water is lower than the near-infrared absorption rate of the catheter or the wires, by using near-infrared light having a shorter wavelength (a wavelength of 1200 nm or the like in the configuration of the present embodiment), increasing the light amount by adjusting the light source using the light amount adjustment device attached to the camera, or the like, or adjusting the shading balance of the observation image with the camera-attached image processing device, the degree of visualization of the lumen region on the observation image was weakened or completely disappeared without almost affecting the fluoroscopic image of the catheter.

According to this method, images at the time of administration and at the time of non-administration of a contrast medium in an X-ray observation image at the time of actual surgery can be easily and instantaneously selectively reproduced without performing a physical operation on an observation target such as administration of a contrast medium.

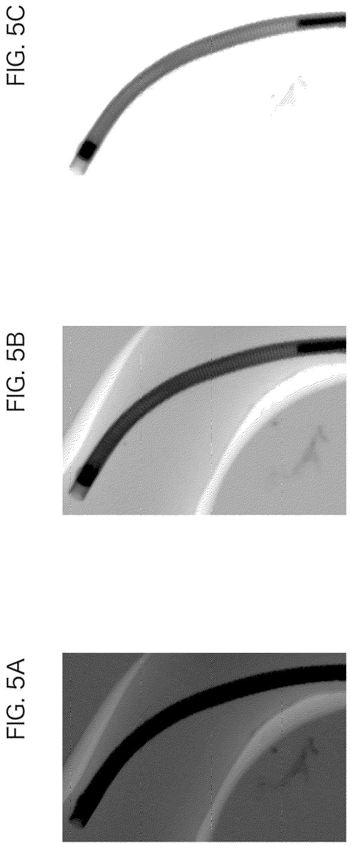

As an example, an observation image when the lumen region is selectively visualized and disappeared by adjusting the light amount is illustrated in FIG. 5. FIG. 5(B) is an observation image obtained when the diaphragm of the accompanying lens of the camera 15 was set to F2.8 using the light source 11 of 1450 nm (the light amount was set to the maximum by the adjustment device accompanying the light source). Under this condition, the lumen region (wall cross-sectional region) of the blood vessel model is visualized in a distinguishable state together with the fluoroscopic image of the catheter. FIG. 5(C) illustrates an observation image obtained when the diaphragm of the accompanying lens of the camera 15 was set to F1.8 while the same light source 11 was used. Under this condition, the fluoroscopic image of the catheter is observed, but the lumen region (wall cross-sectional region) of the blood vessel model has disappeared. FIG. 5(A) illustrates an observation image obtained when the diaphragm of the accompanying lens of the camera 15 was set to F5.0 while the same light source 11 was used. However, under such a condition, the entire observation image becomes dark, and it is difficult to visually recognize the state inside the catheter, which is not appropriate as the observation condition.

Depending on the amount of water, the thickness and the material of the sheath 2 of the catheter 1, and the presence or absence of particles that cause Rayleigh scattering, the conditions of the amount of light that can be obtained in FIGS. 5(B) and 5(C) are changed. For example, in a case where the diameter of the blood vessel model is large, or in a case where the depth of the immersion liquid 7 around the blood vessel model is large, at a wavelength of 1300 nm to 1550 nm, the amount of infrared rays absorbed by water is excessively large, and the obtained image becomes dark as a whole, so that a high light amount was required for the light source.

In order to lower the infrared absorption rate by water and clearly visualize the blood vessel shape, near-infrared light having a wavelength shorter than 1300 nm (near-infrared light having a wavelength of 780 nm to 1250 nm, etc.) can be used. In this case, it is effective to create a state suitable for near-infrared fluoroscopic observation and near-infrared spectroscopic observation by adjusting the light amount of near-infrared light so that the contrast value of only the region containing water decreases.

At the time of simulation of catheter treatment, it is necessary to adjust observation conditions such as a light amount and a wavelength of a light source while observing an image reflected on a display each time according to a catheter or a blood vessel model to be used.

In a case where the height difference of the observation target is large, such as a case where the blood vessel model is three-dimensionally bent, the light amount and the focus may be adjusted according to the distance from the light source to the observation target.

As described above, by near-infrared spectroscopic observation and near-infrared fluoroscopic observation, the observation image of the blood vessel model that can identify the lumen region of the blood vessel model and the fluoroscopic image of the catheter can be simultaneously observed.

On the other hand, by mixing particles interfering with near-infrared light in the material forming the blood vessel model and/or the liquid filling the blood vessel model, it is also possible to enhance the impermeability of the portion where the particles exist at the time of observation with near-infrared light and forcibly visualize the region including the particles, that is, the lumen region of the blood vessel model. As the particles that interfere with near-infrared light, particles that absorb near-infrared light, particles that scatter near-infrared light, or particles that reflect near-infrared light can be used, and for example, aluminum fine particles, India ink (carbon-based fine particles), or the like can be used. Note that, in the near-infrared region, in a case where the particle size is larger than about 100 nm, Mie scattering occurs, the observation image becomes unclear, the permeability is greatly reduced, and therefore such a case is not preferable. In a case where fluoroscopic observation is required, it is preferable to maintain the sharpness of the observation image by causing Rayleigh scattering using particles having a particle diameter of less than 100 nm, more preferably about 0.5 nm to 50 nm, and to exhibit the effect of enhancing the near-infrared fluoroscopic observation ability as compared with the case where no particles are added for the above reason.

Note that particles larger than 100 nm can be blended in a region where fluoroscopic observation of the inside thereof is unnecessary, such as a pseudo thrombus installed in a lumen region of a blood vessel model, and used to visualize said region.

In the above description, the light source is not particularly limited as long as it can emit near-infrared light having an intensity capable of observing a fluoroscopic image of the catheter. From the viewpoint of near-infrared spectroscopic observation, it is possible to adjust the shading balance of each part of the observation target by allowing a plurality of light sources having different wavelengths to coexist or preparing a light source having a variable wavelength.

The selection of the wavelength can also be performed on the camera side. A filter having wavelength selectivity can be installed in front of the camera, or a wavelength can be selected at the time of image processing after light is collected by the camera.

In a case where light sources having different wavelengths are used, an image can be formed by switching the wavelengths. Furthermore, it is also possible to simultaneously form an image corresponding to each wavelength by simultaneously emitting light from light sources having different wavelengths and preparing a camera and a display thereof corresponding to each wavelength.

By allowing the first light source that emits near-infrared light having a wavelength of 1300 nm to 1550 nm having a high absorptivity with water and the second light source having a wavelength of 780 nm to 1250 nm having a relatively low absorptivity with water to coexist, it is possible to selectively visualize or eliminate only the lumen region while maintaining the fluoroscopic image of the catheter with a simple switching operation. Alternatively, the wavelength can be selected so as to generate an appropriate shade according to the diameter of the blood vessel model and the depth of the surrounding immersion liquid. Alternatively, a wavelength exhibiting optimal absorption characteristics or transmission characteristics can be selected according to the type and material of the catheter to be used.

As the light source, it is preferable to employ a surface light source that uniformly irradiates a desired region of the blood vessel model to be observed with near-infrared light. A parallel light source may be used for the purpose of clearly observing the outline of the observation target, or a diffusion light source may be used for the purpose of facilitating the fluoroscopic observation by irradiating the observation target with light from multiple directions. Furthermore, only a partial region of the observation target can be observed using a line light source or a point light source, or a desired region of the blood vessel model can be uniformly irradiated with near-infrared light from the line light source or the point light source via the light diffusion circuit.

The light source can also irradiate the observation target from a plurality of directions. In particular, since near-infrared light have low permeability to metal, in a configuration in which a blood vessel model is arranged between a light source and a camera and observation is performed, in a case where an observation target includes a thick metal structure, near-infrared light is completely blocked by said metal structure. For this reason, when a platinum X-ray opaque marker is partially attached to a stainless delivery wire of the aneurysm embolization coil, it is not possible to identify them.

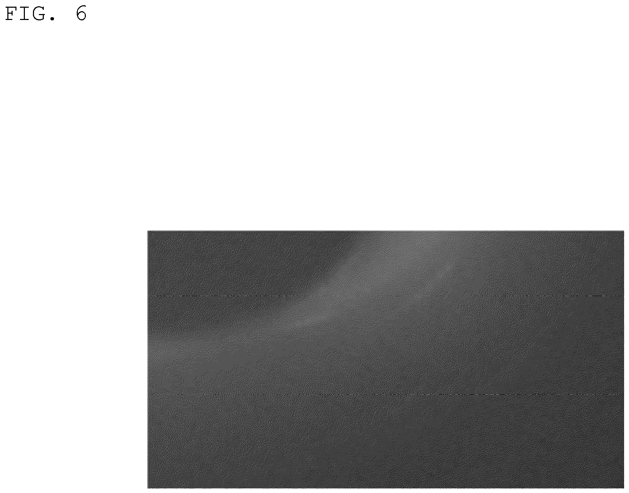

The inventors have found out utilization of the property of near-infrared light which shows different reflectivity depending on the type and wavelength of the metallic material. It has been found that an observation image in which a structure made of a different metal material can be identified as illustrated in FIG. 6, can be obtained by performing observation using reflection of near-infrared light in combination.

In FIG. 6, reference sign 20 denotes the above-mentioned stainless steel delivery wire, and reference sign 30 denotes the above-mentioned platinum X-ray opaque marker. In the near-infrared reflection observation, a second light source for generating reflection of near-infrared light is arranged in a side direction (a direction orthogonal to the axis of the first light source and the camera used for near-infrared fluoroscopic observation, or obliquely upward) of the blood vessel model, and the blood vessel model is irradiated with near-infrared light. A third light source may be added to additionally emit light from a direction different from that of the first and second light sources. In the near-infrared reflection observation, the wavelengths of the first light source, the second light source, and the third light source can be arbitrarily selected, but in order to suppress the chromatic aberration, it is preferable to make the wavelengths of the respective light sources the same.

The camera is not particularly limited as long as it can receive near-infrared light from the light source. A plurality of cameras can be adopted according to the light source.

The display images the output from the camera. Preferably, a light source or a light amount adjustment device attached to the camera is disposed at a position where the light amount can be adjusted while the display is visually recognized so that the light amount incident on the camera can be adjusted while the image on the display is visually recognized.

Studies have been made so that an image of the X-ray opaque marker can be more clearly captured.

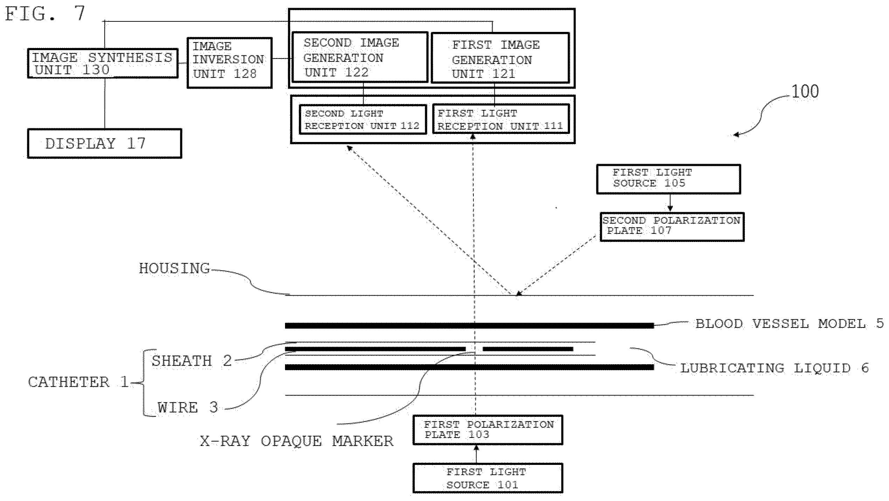

FIG. 7 illustrates a configuration of an image capturing device 100. In FIG. 7, the same elements as those in FIG. 2 are denoted by the same reference signs, and the description thereof will be omitted.

The first near-infrared light having a wavelength of 940 nm is emitted from the first light source of the image capturing device 100, and is polarized in the first direction in a first polarization plate 103. The first near-infrared light thus polarized are received by a first light reception unit 111.

The second near-infrared ray having a wavelength of 850 nm is emitted from the second light source 105, and is polarized in the second direction in a second polarization plate 107. The second near-infrared light thus polarized are reflected by the observation target and received by a second light reception unit 112.

The first image formation unit 121 forms a first image by first near-infrared light received by the first light reception unit 111. The first image formation unit 121 includes a gray-scaled image generation unit, and the first image is gray-scaled.

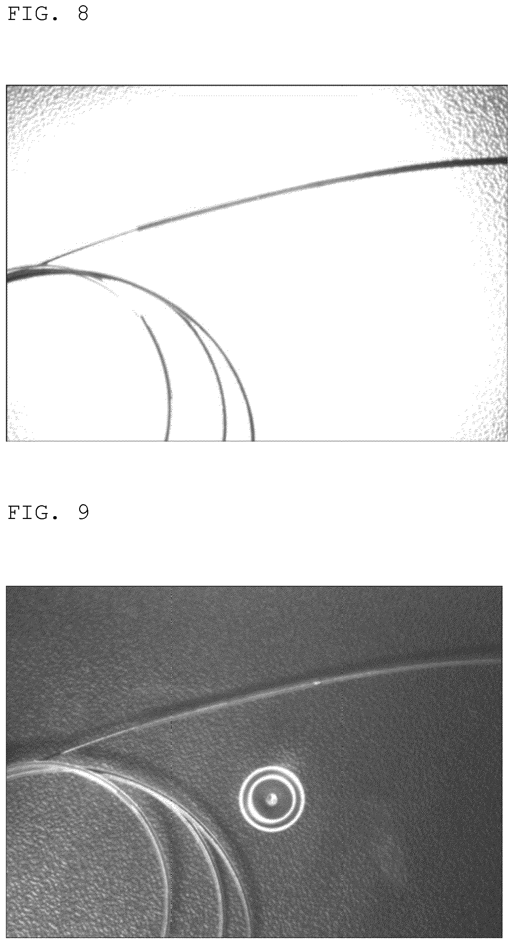

FIG. 8 illustrates an example of the first image.

The second image formation unit 122 forms a second image by second near-infrared light received by the second light reception unit 112. The second image formation unit 122 includes a gray-scaled image generation unit, and the second image is gray-scaled.

FIG. 9 illustrates an example of the second image.

The color tone of the second image is inverted in an image inversion unit 128. Here, inversion means that the value of the gradation of each pixel is inverted about the center value of the grayscale. For example, in the case of the grayscale of 265 gradations, if the value is 160 (= 133 + 27) about the center value 133, the value becomes 106 (= 133 - 27) after inversion.

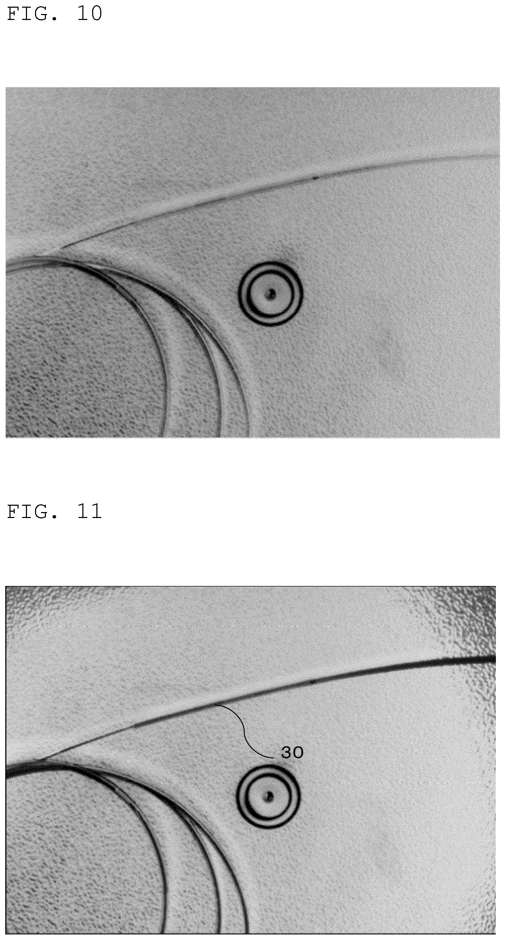

FIG. 10 illustrates an image 2-1 thus inverted.

The image 2-1 and the first image are synthesized in the image synthesis unit 130. In the composition here, the values of the corresponding pixels in both images are numerically multiplied. As a result, black is more emphasized on the black side, and white is more emphasized on the white side.

Note that the alignment between the image 2-1 and the first image is performed by a known method.

An image synthesized in this manner is illustrated in FIG. 11.

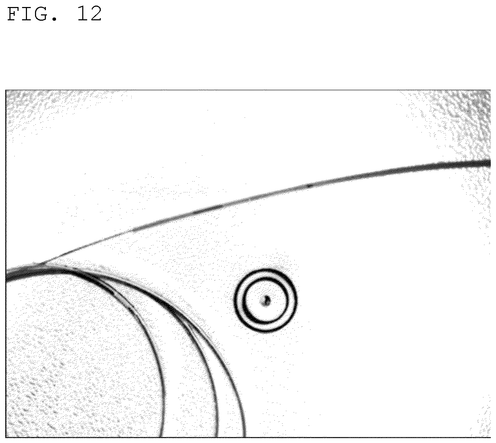

In FIG. 12, the position of the X-ray opaque marker is emphasized by performing density correction.

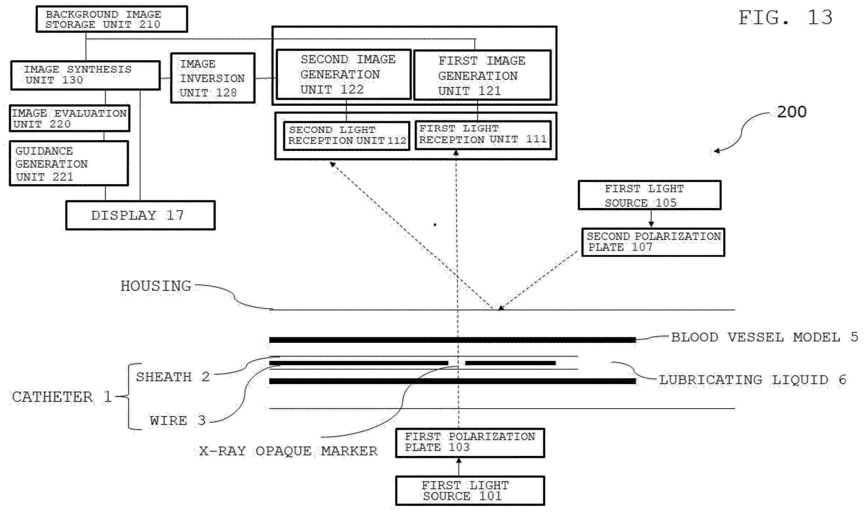

FIG. 13 illustrates an observation device 200 according to another embodiment. In FIG. 13, the same elements as those in FIG. 7 are denoted by the same reference signs, and the description thereof will be omitted.

The observation device 200 in FIG. 13 includes a background image storage unit 210, an image evaluation unit 220, and a guidance generation unit 221.

The background image storage unit 210 stores images of bones and other tissues, and this image can be displayed together with the composite image by the image synthesis unit 130.

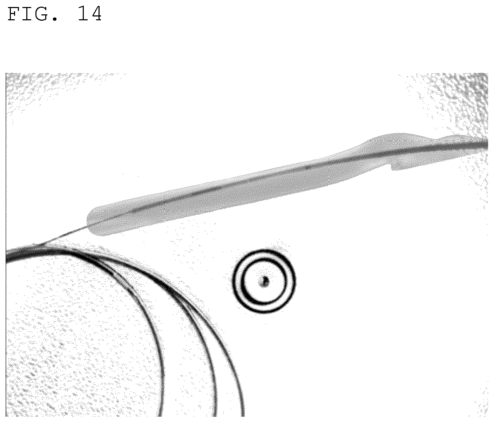

The image evaluation unit 220 evaluates the composite image illustrated in FIG. 12 using, for example, a photoelastic stress observation method or AI. As illustrated in FIG. 14, when the stress generated in the blood vessel model is evaluated by the photoelastic stress observation method, the guidance display unit 221 displays the stress distribution as color (gradation) information superimposed on the composite image as the guidance. At the same time, text information can be also displayed.

In the actual catheter intravascular surgery, blood vessels are only visualized for a few seconds because the administered contrast medium flows away by the blood flow. Furthermore, there is an upper limit to the amount of the contrast medium used due to toxicity, and the contrast medium cannot be administered repeatedly. For this reason, a method (blood vessel roadmap method) is used in which a blood vessel visualization image at the moment when a contrast medium is administered is stored as a still image and superimposed on an observation image in which only a catheter is visualized.

In the near-infrared observation in the present invention, it has been confirmed that the above-described blood vessel road map method can be reproduced by performing image processing in which after a still image in a state in which a blood vessel lumen portion is visualized is stored, an image of the blood vessel lumen portion is eliminated by changing observation conditions to make only a catheter in a visualized state, and the still images are superimposed and presented as necessary. In reproducing the blood vessel road map method, it is preferable that a hardware interface such as a foot pedal and a joystick are further provided in the observation device of the present invention so that various operations associated with the image processing can be performed in a form similar to the actual catheter intravascular surgery.

As the blood vessel model, a cross-linked soft polymer material such as silicone rubber, urethane rubber, soft polyvinyl chloride, and a soft photocurable resin, a hard polymer material such as an acrylic resin and a hard photocurable resin, or a material obtained by combining these materials and formed of a material that transmits near-infrared light can be used. In the lumen region of the blood vessel model, it is necessary to dispose a substance exhibiting infrared absorption characteristics different from that of the forming material of the blood vessel model so that the region can be identified at the time of near-infrared spectroscopic observation. For example, when silicone rubber is used as a material for forming the blood vessel model, the lumen of the blood vessel model can be filled with a lubricating liquid containing water as a main component, or the lumen can be filled with air after the lubricating liquid is applied to the lumen surface of the blood vessel model.

As the blood vessel model in the present invention, a blood vessel model having a membrane-like structure similar to that of a biological blood vessel (hereinafter, referred to as a "membranous blood vessel model"), a blood vessel model in which a cavity simulating a blood vessel lumen is formed inside a block body such as a rectangular parallelepiped, a cylinder, or a sphere (hereinafter, referred to as a block-shaped blood vessel model), or a blood vessel model combining both of them can be used. In the membranous blood vessel model, a liquid, a gas, or a solid (in particular, a gel-like solid) can be selectively disposed in each of the lumen region and the peripheral region of the blood vessel model depending on the situation (for example, water may be placed in the lumen region and water or air may be placed in the surrounding region). As a result, in the membranous blood vessel model, not only the lumen region of the blood vessel model but also the wall cross-sectional region of the blood vessel model can be visualized so as to be distinguishable. In the block-shaped blood vessel model, various substances can be selectively disposed in the lumen region according to the situation.

As a constituent material of such a blood vessel model, various materials such as soft materials such as silicone rubber, urethane rubber, soft polyvinyl chloride and soft photocurable resin, gels made of polyvinyl alcohol and the like, acrylic resin and hard photocurable resin can be used as long as the material has near-infrared light permeability.

Since near-infrared light do not damage human eyes, it is preferable that the polymer material has permeability to visible light so that an operator can visually observe the blood vessel model in a state of being irradiated with near-infrared light.

Examples of the crosslinked polymer material include crosslinked soft polymer materials such as silicone rubber, urethane rubber, soft polyvinyl chloride, and soft photocurable resin, hard polymer materials such as acrylic resin and hard photocurable resin, and materials obtained by combining these materials. The material for forming the blood vessel model, the liquid filling the lumen portion of the blood vessel model, and the like may be colored with a dye or the like that can be identified in a visible light region. Since most of the dyes used in the visible light region exhibit high permeability in the near-infrared region, they hardly affect the near-infrared spectroscopic observation image, and an image observation condition in the visible light region and an image observed in the near-infrared region can be set separately.

A blood vessel model can also be formed from a gel substance such as polyvinyl alcohol. Since the blood vessel model made of polyvinyl alcohol has high lubricity due to its physical properties, the material filled in the lumen of the blood vessel model may be only water or air. Of course, addition of a chemical agent for enhancing lubricity such as a surfactant is not hindered.

A lesion model simulating a thrombus or a plaque can be attached to the blood vessel model in advance or can be movably installed. In performing the near-infrared spectroscopic observation in the present invention, in order to selectively enhance the visibility of the lesion model, it is preferable to form said lesion model using a material exhibiting near-infrared light absorption characteristics different from the formation material of the blood vessel model or the material disposed in the lumen region. By observing the simulation process of catheter vasodilation or thrombus recovery using the plaque or thrombus made of such a material, it is possible to clearly observe a state in which the plaque is crushed by the balloon or the stent in the lumen of the blood vessel model, a state in which the thrombus is captured by the stent and collected while moving in the blood vessel together with the stent, and the like.

Furthermore, in the present invention, the first near-infrared light transmitted through the observation target and the second near-infrared light reflected by the observation target are simultaneously received by using one light reception unit, and an image can be directly formed without performing any calculation based on the received light. In formation of such an image, when the first near-infrared light and the second near-infrared light have different wavelengths, it is preferable that the lens provided in the light reception unit has a focus shift correction function.

In a case where one light reception unit is used, it is also possible to receive the first near-infrared light transmitted through the observation target and the second near-infrared light reflected by the observation target with a time difference, and synthesize them by calculating images generated from respective lights. In this case, the color tone of the second image generated from the second near-infrared light can be inverted, and this inverted second image and the first image generated from the first near-infrared light can be synthesized by calculating those images.

The present invention is not limited to the description of the embodiments and examples of the invention. Various modifications that can be easily conceived by those skilled in the art without departing from the scope of the claims are also included in the present invention.

### REFERENCE SIGNS LIST

- 1: catheter
- 2: sheath
- 3: wires
- 5: blood vessel model
- 10: observation device
- 20: wire
- 30: marker

## Claims

1. An observation device that observes a blood vessel model as an observation target into which a catheter is inserted, the blood vessel model including a substance disposed inside having near-infrared light absorption characteristics different from that of a constituent material of the blood vessel model, and the catheter including a sheath and wires inserted into the sheath, the observation device comprising:
a first light source that emits first near-infrared light; a first light reception unit that can receive the first near-infrared light; a first image generation unit that generates a first image based on the first near-infrared light captured by the first light reception unit; and a display that displays the first image.

2. An observation method of an observation target using the observation device according to claim 1, the observation method comprising:
causing the first light source to emit the first near-infrared light toward the observation target, and causing the first light reception unit to receive the first near-infrared light transmitted through the observation target;
causing the first image generation unit to generate the first image based on the first near-infrared light received by the first light reception unit;
causing the display to display the first image; and
adjusting a light amount of the first near-infrared light used for generating the first image as necessary to enable simultaneous visual recognition of the sheath of the catheter, the wires in the sheath, and the blood vessel model.

3. The observation device according to claim 1, further comprising: a second light source that emits second near-infrared light; a second light reception unit that can receive the second near-infrared light; a second image generation unit that generates a second image based on the second near-infrared light captured by the second light reception unit; and
an image synthesis unit that superimposes the first image and the second image,
wherein the first image and the second image are displayed overlaid on the first image on the display.

4. An observation method of an observation target using the observation device according to claim 3, the observation method comprising:
causing the first light source to emit the first near-infrared light toward the observation target, and causing the first light reception unit to receive the first near-infrared light transmitted through the observation target;
causing the first image generation unit to generate the first image based on the first near-infrared light received by the first light reception unit;
causing the display to display the first image;
causing the second light source to emit the second near-infrared light toward the observation target, and causing the second light reception unit to receive the second near-infrared light reflected by the observation target;
causing the second image generation unit to generate the second image based on the second near-infrared light received by the second light reception unit;
causing the image synthesis unit to generate a composite image in which the first image and the second image are superimposed;
causing the display to display the composite image; and
adjusting a light amount of the first near-infrared light used for generating the first image as necessary, and/or adjusting a light amount of the second near-infrared light used for generating the second image as necessary to enable simultaneous visual recognition of the sheath of the catheter, the wires in the sheath, an X-ray opaque marker of the wires, and the blood vessel model.

5. The observation device according to claim 3, further comprising:
a first polarization section that polarizes the first near-infrared light in a first direction;
a second polarization section that polarizes the second near-infrared light in a second direction;
a grayscale image generation unit that gray-scales the first image and the second image; and
a gradation inversion unit that generates an image 2-1 by inverting gradation of a grayscale image of the second image.

6. An observation method of an observation target using the observation device according to claim 5, the observation method comprising:
causing the first image generation unit to generate the first image based on the first near-infrared light polarized in the first direction;
causing the second image generation unit to generate the second image based on the second near-infrared light polarized in the second direction;
causing the grayscale image generation unit to grayscale the first image and the second image;
causing the gradation inversion unit to invert gradation of the gray-scaled second image to form an image 2-1, and
causing the image synthesis unit to superimpose the gray-scaled first image and the image 2-1, and synthesize them by calculating those value of each pixel on the images.

7. The observation method according to claim 4, wherein density correction is performed to emphasize the X-ray opaque marker.

8. The observation device according to claim 3, further comprising:
an image evaluation unit that evaluates a composite image synthesized by the image synthesis unit; and
a guidance formation unit that forms guidance based on an evaluation result of the image processing unit,
wherein the guidance is displayed on the display.

9. An observation method of an observation target using the observation device according to claim 8, the observation method comprising:
causing the image evaluation unit to evaluate the composite image;
causes the guidance formation unit to form the guidance based on an evaluation result of the image evaluation unit; and
causing the display to display the guidance.

10. An observation device that observes a blood vessel model as an observation target into which a catheter is inserted, the blood vessel model including a substance disposed inside having near-infrared light absorption characteristics different from that of a constituent material of the blood vessel model, and the catheter including a sheath and wires inserted into the sheath, the observation device comprising:
a first light source that emits first near-infrared light; a first light reception unit that can receive the first near-infrared light transmitted through the observation target; a first image generation unit that generates a first image based on the first near-infrared light captured by the first light reception unit; and a display that displays the first image,
wherein a light amount of the first near-infrared light used for generating the first image is adjusted.

11. An observation device that observes a blood vessel model as an observation target into which a catheter is inserted, the blood vessel model including a substance disposed inside having near-infrared light absorption characteristics different from that of a constituent material of the blood vessel model, and the catheter including a sheath and wires inserted into the sheath, the observation device comprising:
a second light source that emits second near-infrared light; a second light reception unit that can receive the second near-infrared light reflected from the observation target; a second image generation unit that generates a second image based on the second near-infrared light captured by the second light reception unit; and a display that displays the second image,
wherein a light amount of the second near-infrared light used for generating the second image is adjusted.

12. The observation device according to claim 3, wherein the first near-infrared light and the second near-infrared light include monochromatic light.

13. The observation method according to claim 4, further comprising including a material that receives near-infrared light from the light source and causes Rayleigh scattering, in the substance having near-infrared light absorption characteristics different from that of the constituent material of the blood vessel model.

14. The observation method according to claim 4, further comprising including a material that receives near-infrared light from the light source and causes Rayleigh scattering, in the constituent material of the blood vessel model.
